# FASCICULE DE BREVET EUROPEEN

(11) **EP 0 876 210 B1**
(45) Date de publication et mention de la délivrance du brevet: **25.04.2001**
(21) Numéro de dépôt: 96942424.1
(22) Date de dépôt: 18.12.1996
(51) Int. Cl.: B01J 23/22, B01J 23/18, C07C 253/24

(54) **PROCEDE DE PREPARATION DE CATALYSEURS D'AMMOXYDATION POUR REACTEUR A LIT FLUIDISE OU A LIT TRANSPORTE**
VERFAHREN ZUR HERSTELLUNG VON AMMOXIDATIONS-KATALYSATOREN FÜR WIRBELBETT ODER WANDERBETT-REAKTOREN
METHOD FOR PREPARING AMMOXIDATION CATALYSTS FOR A FLUIDISED-BED OR MOVING-BED REACTOR

(30) Priorité: 22.12.1995 FR 9515783
(43) Date de publication de la demande: 11.11.1998
(73) Titulaire: RHODIA POLYAMIDE INTERMEDIATES, 69190 Saint-Fons (FR)
(72) Inventeur: BLANCHARD, Gilbert, F-60330 Le Plessis-Belleville (FR); BURATTIN, Paolo, F-69002 Lyon (FR); CAVANI, Fabrizio, I-41100 Modena (IT); MASETTI, Stéfano, I-40137 Bologna (IT); TRIFIRO, Ferruccio, I-40136 Bologna (IT)
(74) Mandataire: Esson, Jean-Pierre
(86) Numéro de dépôt international: FR9602022
(87) Numéro de publication internationale: WO9723287

(56) Documents cités:
- EP-A- 0 037 123
- EP-A- 0 059 414
- EP-A- 0 558 424
- WO-A-95/05895

## Description

La présente invention concerne un procédé de préparation d'oxydes mixtes à base de vanadium, d'antimoine et éventuellement d'étain et/ou de titane et/ou de fer et/ou d'autres métaux, déposés sur un support ainsi que leur utilisation comme catalyseurs pour l'ammoxydation des alcanes.

Certains oxydes mixtes de vanadium et d'antimoine ou de vanadium, d'antimoine et d'autres métaux sont des composés connus qui ont été décrits, parmi de nombreux autres oxydes mixtes, dans le brevet FR-A-2 072 334.

Dans le brevet US 5 008 427, il est décrit un procédé d'ammoxydation du propane ou du butane, en présence d'un catalyseur pouvant comprendre notamment des oxydes de vanadium, d'antimoine et de fer ou de titane ou de chrome ou de gallium ou d'étain, ainsi qu'éventuellement d'autres métaux. Ces catalyseurs présentent la caractéristique essentielle d'avoir été calcinés à une température égale ou supérieure à 780°C.

De même, dans le brevet EP-A-0 558 424, il a été décrit un procédé d'ammoxydation d'alcanes, catalysé par des oxydes mixtes de vanadium, d'antimoine, de fer et/ou de gallium et/ou d'indium. La préparation de ces oxydes mixtes se fait par mélange de suspensions aqueuses de composés des différents métaux, chauffage sous agitation, puis évaporation de l'eau, séchage et calcination.

Ces brevets ne présentent pas d'indication particulière concernant la technologie de réacteur associée aux catalyseurs d'ammoxydation des alcanes qu'ils décrivent.

Cependant pour le type de réaction représenté par l'ammoxydation des alcanes, il s'avère que l'utilisation de réacteur à lit fluidisé ou à lit transporté est plus avantageuse. En raison de l'importante exothermicité des réactions intervenant dans l'ammoxydation des alcanes, la possibilité de mettre en oeuvre, à l'échelle industrielle, un ou plusieurs réacteurs à lit fixe (réacteurs multitubulaires) paraît peu réaliste, voire exclue, en particulier si l'on veut atteindre des productivités élevées. En effet pour éliminer les calories dégagées, il faudrait utiliser un réacteur multitubulaire de très grande taille ou toute une batterie de réacteurs multitubulaires, ce qui est rédhibitoire au plan des investissements.

Par rapport au réacteur à lit fixe, les réacteurs à lit fluidisé ou transporté possèdent une plus grande capacité à évacuer les calories dégagées et paraissent de ce fait mieux adaptés à la réaction d'ammoxydation des alcanes. Cette meilleure capacité à transférer la chaleur permet d'envisager un gain de productivité par l'augmentation de la teneur en alcane dans le mélange d'alimentation. Une telle opération ne peut être envisagée dans le cas d'un réacteur à lit fixe, car les limitations au niveau du transfert de chaleur (qui se manifestent par l'existence d'un point chaud dans le réacteur) imposent l'utilisation de relativement faibles teneurs en alcane, de manière à éviter tout problème d'explosivité ou d'inflammabilité du mélange gazeux.

La présente invention concerne un procédé de préparation d'un catalyseur pour réacteur à lit fluidisé ou à lit transporté comprenant une phase active de formule empirique générale (I) :

V Sbₐ Sn_{b} Ti_{c} Fe_{d} Eₑ Oₓ (I)

dans laquelle :
- E représente un élément pouvant donner un oxyde de structure rutile ou un élément qui, associé à V, Sb, Sn, Ti, Fe et/ou à un autre élément E, peut donner une phase de structure rutile ou trirutile ou une solution solide de structure rutile
- a représente un nombre entier ou fractionnaire égal ou supérieur à 0,5
- b, c, d et e représentent indépendamment les uns des autres un nombre entier ou fractionnaire de 0 à 100
- x représente un nombre entier ou fractionnaire déterminé par le degré d'oxydation des autres éléments,
   et un support solide de type oxyde,
ledit procédé étant caractérisé en ce qu'il comprend :
- l'imprégnation du support solide par une solution dans au moins un alcool saturé de composés respectifs de vanadium, d'antimoine et éventuellement d'étain et/ou de titane et/ou de fer et/ou d'élément E,
- la mise en contact du support solide imprégné obtenu, avec une solution aqueuse tampon à pH compris entre 6 et 8,
- la séparation du solide et son séchage,
- la calcination du solide en deux étapes, tout d'abord à une température de 300°C à 350°C, puis à une température de 400°C à 800°C.

Les éléments E sont notamment choisis parmi le germanium, le manganèse, le ruthénium, le niobium, le tantale, le gallium, le chrome, le rhodium, le nickel, le molybdène, l'aluminium, le thorium, le calcium, le tungstène et le magnésium. Il peut y avoir plusieurs éléments E dans la formule (I) et dans le présent texte l'expression élément E couvre un ou plusieurs éléments E.

Les composés du vanadium, de l'antimoine, de l'étain, du titane, du fer et de l'élément E mis en oeuvre dans le procédé doivent être solubles dans un alcool saturé ou un mélange d'alcools saturés.

Dans le présent texte, on considère qu'un composé est soluble lorsque sa solubilité mesurée à 25 °C est d'au moins 5 grammes par litre d'alcool saturé ou d'eau. Ces composés peuvent être introduits ensemble ; ils peuvent aussi tout d'abord être mis séparément en solution dans un alcool, les différentes solutions alcooliques ainsi obtenues étant ensuite mélangées entre elles. Généralement, mais non limitativement, on prépare une solution alcoolique par dissolution des différents composés, sans préparation intermédiaire de solutions de chacun des composés du vanadium, de l'antimoine et le cas échéant de l'étain, du titane, du fer et de l'élément E.

A titre d'exemples de composés solubles du vanadium, on peut citer l'acétylacétonate de vanadyle, le trichlorure de vanadyle, le trifluorure de vanadium, le tétrafluorure de vanadium, le pentafluorure de vanadium, le tribromure de vanadium, le dichlorure de vanadium, le trichlorure de vanadium, le tétrachlorure de vanadium, le triiodure de vanadium.

A titre d'exemples de composés solubles de l'antimoine, on peut citer le pentachlorure d'antimoine, le trichlorure d'antimoine, le tribromure d'antimoine, le trifluorure d'antimoine, le triiodure d'antimoine, le trioxyde d'antimoine, l'hydrure d'antimoine.

A titre d'exemples de composés solubles de l'étain, on peut citer le chlorure stannique, le chlorure stanneux, le bromure stanneux.

A titre d'exemples de composés solubles du titane, on peut citer le dichlorure de titane, le tétrachlorure de titane, le trichlorure de titane, le tribromure de titane, le tétrabromure de titane, le tétrafluorure de titane, le diiodure de titane.

A titre d'exemples de composés solubles du fer, on peut citer le dichlorure de fer, le trichlorure de fer, le dibromure de fer, le tribromure de fer, le diiodure de fer, le nitrate ferreux, le sulfate ferreux, le sulfate ferrique, le thiosulfate ferreux, le formiate ferrique, l'acétate ferrique, l'acétylacétonate ferrique, le benzoate ferrique, l'oléate ferrique, le lactate ferreux, le lactate ferrique.

Les alcools saturés mis en oeuvre dans le procédé de l'invention sont plus particulièrement les alcanols et les cycloalcanols. De préférence, on utilisera les alcanols et cycloalcanols dont la température d'ébullition ne soit pas trop élevée, afin de faciliter les opérations de séparation ou de recyclage par distillation ou évaporation. Ainsi les alcanols ayant de 1 à 6 atomes de carbone, tels que le méthanol, l'éthanol, le n.propanol, le propanol-2, le n.butanol, le butanol-2, le tertiobutanol, les pentanols et les hexanols, et le cyclohexanol sont préférés.

Le support solide est un oxyde tel qu'une alumine, une silice, une silice-alumine, une zircone, une cérine, un oxyde mixte de cérium et de zirconium, une magnésie, un oxyde de titane, un oxyde de niobium, un oxyde de lanthane.

La taille des particules du support solide est généralement de 10 µm à 1000 µm et de préférence de 20 µm à 300 µm.

Une autre caractéristique importante du support solide est sa capacité d'être imprégné par une solution des composés des métaux de la phase active. Ainsi, on utilisera les supports ayant un volume poreux total d'au moins 0,1 cm³/g et de préférence d'au moins 0,15 cm³/g.

La qualité de la fluidisation du lit catalytique peut dépendre aussi d'autres paramètres tels que notamment la densité du support, le taux de vide du support, le débit gazeux. La fluidisation souhaitée du catalyseur de l'invention, en particulier pour l'application en ammoxydation des alcanes, sera effectuée de manière optimale par l'homme du métier en tenant compte de ces divers paramètres.

Parmi les supports solides présentant les caractéristiques indiquées précédemment, on préfère utiliser les silices dans le procédé selon l'invention.

L'imprégnation du support solide par la solution alcoolique des composés des métaux entrant dans la composition de la phase active de formule (I) est effectuée en mettant en oeuvre un volume de solution alcoolique inférieur ou égal au volume poreux total du support. Lorsque le volume de la solution alcoolique est inférieur au volume poreux total du support, il représentera au moins 50 % dudit volume poreux total. La durée de la phase d'imprégnation n'est pas critique. Elle peut simplement varier en pratique selon le mode de mélange du support et de la solution qui est choisi.

Le support solide imprégné est ensuite mis en contact avec une solution aqueuse tampon ayant un pH compris entre 6 et 8 et de préférence compris entre 6,5 et 7,5, ladite solution tampon étant de préférence une solution aqueuse d'un sel d'ammonium, comportant éventuellement de l'ammoniaque. Le sel d'ammonium utilisé est plus préférentiellement un carboxylate d'ammonium (par exemple acétate, citrate, tartrate), l'oxalate d'ammonium, le carbonate d'ammonium, l'hydrogénophosphate d'ammonium, permettant d'avoir un pH proche de 7, éventuellement en présence d'ammoniaque. Ainsi les solutions tampons acétate d'ammonium/ammoniaque conviennent particulièrement bien.

Le volume de la solution tampon mise en contact avec le support imprégné est égal ou supérieur au volume poreux total du support et est de préférence supérieur au volume poreux total du support.

Le solide obtenu est séparé du liquide par les moyens habituellement utilisés pour cette opération, par exemple par filtration ou par centrifugation.

Il est ensuite séché à une température permettant d'éliminer l'eau et dépendant de la pression sous laquelle on opère. Si l'on opère sous pression atmosphérique, la température de séchage sera avantageusement de 100°C à 200°C, de préférence de 110°C à 180°C. Si l'on opère sous pression inférieure à la pression atmosphérique, la température de séchage peut être plus faible, généralement égale ou supérieure à 50°C.

La durée du séchage peut varier largement selon la température choisie. Elle sera généralement déterminée de façon à éliminer la majeure partie de l'eau d'imprégnation. Elle se situe le plus souvent entre quelques minutes et quelques dizaines d'heures.

La calcination du solide ainsi séché est effectuée dans un four à une température de 300°C à 350°C, puis à une température de 400°C à 800°C, et plus préférentiellement entre 500°C et 750°C pour la deuxième étape, et pendant des durées variant de manière indicative de quelques minutes à plusieurs heures, le plus généralement de 30 minutes à 20 heures.

Dans le catalyseur final, le rapport pondéral entre la phase active de formule (I) et la totalité dudit catalyseur est généralement de 5 à 50 %.

Parmi les oxydes mixtes de la phase active de formule (I) définis précédemment, on préfère ceux pour lesquels :
- E représente un ou plusieurs éléments choisis parmi le nickel, le gallium, l'aluminium, le niobium
- a représente un nombre entier ou fractionnaire égal ou inférieur à 100 et compris de préférence entre 0,5 et 50
- b, c, d et e représentent indépendamment les uns des autres un nombre entier ou fractionnaire de 0 à 50, l'un au moins de ces symboles étant supérieur à 0 et de préférence égal ou supérieur à 0,5
- x représente un nombre entier ou fractionnaire déterminé par le degré d'oxydation des autres éléments.

La présente invention a également pour objet un procédé d'ammoxydation d'alcanes en phase vapeur dans un réacteur à lit fluidisé ou à lit transporté, en présence d'un catalyseur comprenant une phase active de formule empirique générale (I) :

V Sbₐ Sn_{b} Ti_{c} Fe_{d} Eₑ Oₓ (I)

dans laquelle :
- E représente un élément pouvant donner un oxyde de structure rutile ou un élément qui, associé à V, Sb, Sn, Ti, Fe et/ou à un autre élément E, peut donner une phase de structure rutile ou trirutile ou une solution solide de structure rutile
- a représente un nombre entier ou fractionnaire égal ou supérieur à 0,5
- b, c, d et e représentent indépendamment les uns des autres un nombre entier ou fractionnaire de 0 à 100
- x représente un nombre entier ou fractionnaire déterminé par le degré d'oxydation des autres éléments,
   et un support solide de type oxyde,
ledit catalyseur procédé étant préparé par le procédé décrit précédemment qui comprend :
- l'imprégnation du support solide par une solution dans au moins un alcool saturé de composés respectifs de vanadium, d'antimoine et éventuellement d'étain et/ou de titane et/ou de fer et/ou d'élément E,
- la mise en contact du support solide imprégné obtenu, avec une solution aqueuse tampon à pH compris entre 6 et 8,
- la séparation du solide et son séchage,
- la calcination du solide en deux étapes, tout d'abord à une température de 300°C à 350°C, puis à une température de 400°C à 800°C.

Selon la présente invention des alcanes ayant de 3 à 12 atomes de carbone par molécule sont mis à réagir en phase vapeur avec de l'ammoniac et de l'oxygène en présence d'un catalyseur dont la composition et la préparation sont indiquées dans la description précédente.

Bien entendu, dans le cadre du présent procédé, on peut utiliser des gaz diluants, inertes dans les conditions réactionnelles tels que l'hélium, l'azote et l'argon. De même, de la vapeur d'eau peut être ajoutée au mélange gazeux réactionnel dans de larges limites. Ainsi le gaz réactif (alcane, ammoniac, oxygène) peut être dilué par un diluant inerte et/ou par de la vapeur d'eau. Dans cet ensemble la teneur volumique en vapeur d'eau peut varier dans de larges limites en particulier de 0 à 50 % et, de préférence entre 3 et 30 %. Pour une bonne mise en oeuvre du procédé selon l'invention, la teneur volumique en gaz réactif sera d'au moins 3 % et de préférence d'au moins 20 %.

Au sein du gaz réactif les teneurs volumiques respectives en alcane, ammoniac et oxygène peuvent varier dans de larges limites.

La teneur en alcane dans le gaz réactif est de préférence comprise entre 5 et 70 %. Celle en ammoniac est de préférence comprise entre 3 et 50 % et celle en oxygène est de préférence comprise entre 3 et 45 %.

En partant de propane, on obtiendra un mélange renfermant essentiellement de l'acrylonitrile. L'acrylonitrile est un intermédiaire industriellement produit sur une large échelle.

En partant d'isobutane, on obtiendra essentiellement du méthacrylonitrile.

Le procédé selon l'invention convient plus particulièrement à l'ammoxydation du propane.

Si l'alcane mis en oeuvre peut être de qualité technique, il ne renfermera pas de quantités importantes de composés à insaturation éthylénique. Ainsi le propane engagé ne renfermera généralement que des quantités mineures de propylène, par exemple inférieures à 10 %.

Comme indiqué précédemment, le procédé selon l'invention est réalisé sous forme de réaction en phase vapeur, dans un réacteur à lit fluidisé ou à lit transporté. Le procédé peut être conduit en continu ou en discontinu.

La température de réaction est en général comprise entre 300°C et 550°C et, de préférence, entre 400°C et 500°C.

La pression totale du mélange réactionnel peut être supérieure ou égale à la pression atmosphérique. Elle est généralement comprise entre 1 et 6 bar et, de préférence entre 1 et 4 bar.

La vitesse volumique horaire est généralement comprise entre 100 et 36000 h⁻¹. Elle dépend du débit gazeux, mais également d'autres paramètres de la réaction. La vitesse volumique horaire se situe de préférence entre 200 et 20000 h⁻¹.

La vitesse volumique horaire se définit comme le rapport volume gazeux total/volume du catalyseur/heure.

Bien entendu, l'homme de l'art sera à même de trouver un compromis entre la température, le débit gazeux, la nature précise du catalyseur mise en oeuvre et les divers autres paramètres de la réaction compte-tenu de ses objectifs de production.

Les produits de la réaction peuvent être récupérés dans les gaz effluents par tout moyen approprié. Par exemple les gaz effluents peuvent passer dans un condenseur contenant de l'acide sulfurique dilué pour neutraliser l'ammoniac non converti. Les gaz peuvent ensuite passer sur une colonne absorbante réfrigérée pour condenser l'acrylonitrile, l'acétonitrile et l'acide cyanhydrique, les vapeurs non condensées contenant principalement du propane non converti, du propylène, des hydrocarbures légers et le cas échéant du CO₂. On peut ensuite séparer l'acrylonitrile et l'acide cyanhydrique de l'acétonitrile par distillation, puis distiller à son tour le mélange acrylonitrile-acide cyanhydrique récupéré pour séparer l'acrylonitrile de l'acide cyanhydrique.

Les exemples ci-après illustrent la présente invention.

### EXEMPLES DE PREPARATION D'OXYDES MIXTES.

### EXEMPLE 1 - Préparation de catalyseur (A) selon l'invention comprenant un oxyde mixte de formule empirique : V Sb5 Sn5 Ox et un support silice

On imprègne à sec (c'est-à-dire avec un volume de solution inférieur ou égal au volume poreux du support) 13 g de silice ayant des tailles de particules de 30 à 100 µm, une surface spécifique BET de 320 m²/g, un volume poreux de 1,2 cm³/g et une densité de 0,36, avec 15,6 cm³ d'une solution éthanolique contenant 2,15 g de SnCl₄ 0,437 g d'acétylacétonate de vanadyle et 2,46 g de SbCl₅.

Le solide imprégné est versé dans 150 cm³ d'une solution aqueuse contenant de un tampon d'acétate d'ammonium/ammoniaque (65/15 g/l) et ayant un pH de 7,0.

Le solide est ensuite séparé par centrifugation, séché à 120°C pendant 24 heures.

Il est alors calciné pendant 1 h à 350°C, puis 3 h à 700°C

Le catalyseur ainsi obtenu comprend 17 % en poids d'oxyde mixte V Sb₅ Sn₅ Oₓ et 83 % en poids de support silice, a une surface spécifique de 280 m²/g et une densité de 0,43.

### EXEMPLE 2 - Préparation de catalyseur (B) selon l'invention comprenant un oxyde mixte de formule empirique : V Sb5 Sn5 Ox et un support alumine

On imprègne à sec (c'est-à-dire avec un volume de solution inférieur ou égal au volume poreux du support) 39 g d'alumine ayant des tailles de particules de 20 à 90 µm, une surface spécifique BET de 180 m²/g, un volume poreux de 0,4 cm³/g et une densité de 0,74, avec 15,6 cm³ d'une solution éthanolique contenant 2,15 g de SnCl₄, 0,437 g d'acétylacétonate de vanadyle et 2,46 g de SbCl₅.

Le solide imprégné est versé dans 150 cm³ d'une solution aqueuse contenant de un tampon d'acétate d'ammonium/ammoniaque (65/15 g/l) et ayant un pH de 7,0.

Le solide est ensuite séparé par centrifugation, séché à 120°C pendant 24 heures.

Il est alors calciné pendant 1 h à 350°C, puis 3 h à 700°C

Le catalyseur ainsi obtenu comprend 6,5 % en poids d'oxyde mixte V Sb₅ Sn₅ Oₓ et 93,5 % en poids de support alumine, a une surface spécifique de 150 m²/g et une densité de 0,87.

### EXEMPLES 3 A 5 - Préparation de catalyseurs (C),(D) et (E)selon l'invention comprenant des oxydes mixtes de formules empiriques :

**V Sb2,5 Sn2,5 Ox**
**V Sb5 Sn5 Ox**
**V Sb2,5 Sn2,5 Ox**
**et un support silice**
En utilisant le mode opératoire et les réactifs décrits dans l'exemple 1 et en adaptant le cas échéant les quantités desdits réactifs, on prépare les catalyseurs suivants :
- Catalyseur (C) comprenant 17 % en poids d'oxyde mixte V Sb_{2,5} Sn_{2,5} Oₓ et 83 % en poids de support silice et ayant une surface spécifique de 195 m²/g ;
- Catalyseur (D) comprenant 23 % en poids d'oxyde mixte V Sb₅ Sn₅ Oₓ et 77 % en poids de support silice et ayant une surface spécifique de 235 m²/g ;
- Catalyseur (E) comprenant 23 % en poids d'oxyde mixte V Sb_{2,5} Sn_{2,5} Oₓ et 77 % en poids de support silice et ayant une surface spécifique de 250 m²/g.

### MODE OPERATOIRE GENERAL DES TESTS D'AMMOXYDATION

L'évaluation des performances catalytiques des catalyseurs (A) et (B) a été réalisée dans un réacteur à lit fluidisé en verre, de diamètre intérieur 1,8 cm et de hauteur 20 cm, équipé d'un thermocouple axial, de manière à pouvoir mesurer la température le long du lit catalytique. Environ 15 cm³ de catalyseur sont employés pour chaque test d'ammoxydation ; en raison de la différence de densité entre la silice et l'alumine, ce volume correspond par exemple à 6,4 g pour le catalyseur (A) et à 13,0 g pour le catalyseur (B).

Le mélange réactionnel utilisé dans la série de tests effectuée sur réacteur à lit fluidisé a la composition volumique suivante : C₃H₈/NH₃/O₂/He = 25/10/20/45.

La pression totale du mélange réactionnel est de 1,3 bar pour chaque exemple.

Le débit total gazeux est réglé de façon à obtenir différents temps de contact ; le temps de contact est calculé en considérant le volume du lit catalytique avant fluidisation (15 cm³) et le débit gazeux dans les conditions standard de température et de pression. Les temps de contact mis en jeu sont les suivants : 5 s (débit total : 180 cm³/min), 10 s (débit total : 90cm³/min) et 15 s (débit total : 60cm³/min).
Les vitesses linéaires correspondantes du gaz (vitesses en fût vide), calculées pour des conditions standard de température et pression et en tenant compte de la présence du thermocouple axial dans le réacteur, sont de 105 cm/min, 52 cm/min et 35 cm/min.

Le principe du test d'ammoxydation du propane est le suivant:
- On porte le catalyseur à une température T₁, par exemple 310°C, et après 30 min de stabilisation à la température T₁, on détermine par chromatographie en phase gazeuse la composition du mélange en sortie de réacteur.
- On calcule les pourcentages de conversion et les sélectivités obtenus sur le catalyseur examiné à la température d'entrée T₁ par des relations du type : conversion du propane (en moles %) = propane transformé/propane introduit sélectivité en acrylonitrile (en moles %) = propane transformé en acrylonitrile/propane converti.
- On porte ensuite le catalyseur de 310 à 550°C par incrément de 20°C et on détermine toutes les 40 min les pourcentages de conversion et les sélectivités.

Dans les exemples d'ammoxydation ci-après, les conventions suivantes sont utilisées :
Temp = température de la réaction
TTC₃H₈ = conversion du propane
SACN = sélectivité en acrylonitrile
SACN+C₃H₆ = sélectivité en acrylonitrile et propylène
SAMMOX = sélectivité en acétonitrile, en acide cyanhydrique et en autres sous-produits d'ammoxydation
tc (s) = temps de contact en secondes.

Le complément à 100 % des sélectivités correspond à la formation de CO et de CO₂ ainsi qu'éventuellement de méthane, d'éthane et d'éthylène.

### EXEMPLES D'AMMOXYDATION DU PROPANE

### EXEMPLES 6 A 10

On réalise l'ammoxydation du propane, comme décrit précédemment, en utilisant les catalyseurs A, B, C, D et E selon l'invention.

On peut préciser, à titre indicatif pour le catalyseur A, que le débit minimal de fluidisation est de 9-10 cm³/min (vitesse en fût vide de 6 cm/min).

L'expansion du lit fluidisé est linéaire jusqu'à 60-70 cm³/min (35-40 cm/min). La hauteur du lit catalytique évolue de 10,8 cm (lit dense non fluidisé pour les débits inférieurs au débit minimal de fluidisation) à 16,5 cm. Pour les débits supérieurs à 70 cm³/min, il n'y a plus de variation de la hauteur du lit catalytique.

Les conditions de température et les résultats obtenus sont rassemblés dans le tableau 1 ci-après.

**Tableau 1**

| Exemples | Catalyseur utilisé | tc (s) | Temp (°C) | TT C3H8 (en %) | SACN (en %) | SACN + C3H6 (en %) | SAMMOX (en %) |
|---|---|---|---|---|---|---|---|
| Exemple 6 | A | 5 | 445 | 3,5 | 34 | 36 | 32 |
| | | | 480 | 5 | 41 | 42 | 25 |
| | | 10 | 455 | 8 | 50 | 51 | 26 |
| | | 15 | 450 | 8 | 41 | 42 | 20 |
| | | | 470 | 11 | 32 | 33 | 14 |
| Exemple 7 | B | 5 | 455 | 18 | 5 | 25 | 16 |
| | | | 490 | 27 | 7 | 28 | 12 |
| | | 10 | 455 | 22 | 3 | 17 | 11 |
| | | | 485 | 22 | 6 | 25 | 15 |
| Exemple 8 | C | 5 | 470 | 9 | 55 | 55 | 14 |
| | | 10 | 450 | 9 | 43 | 43 | 20 |
| | | | 470 | 15 | 45 | 45 | 15 |
| | | 15 | 450 | 15 | 45 | 45 | 26 |
| | | | 470 | 20 | 42 | 42 | 18 |
| Exemple 9 | D | 5 | 470 | 11 | 46 | 46 | 25 |
| | | 10 | 450 | 12 | 41 | 41 | 26 |
| | | | 470 | 13 | 41 | 41 | 21 |
| | | 15 | 430 | 14 | 37 | 37 | 23 |
| | | | 450 | 16 | 39 | 39 | 18 |
| | | | 470 | 23 | 38 | 38 | 14 |
| | | | 490 | 17 | 23 | 23 | 14 |
| Exemple 10 | E | 15 | 390 | 8 | 29 | 29 | 35 |
| | | | 410 | 17 | 26 | 26 | 22 |
| | | | 430 | 24 | 30 | 30 | 16 |
| | | | 450 | 22 | 31 | 31 | 14 |

## Revendications

1. Procédé de préparation d'un catalyseur pour réacteur à lit fluidisé ou à lit transporté comprenant une phase active de formule empirique générale (I) :
V Sbₐ Sn_{b} Ti_{c} Fe_{d} Eₑ Oₓ (I)
dans laquelle :
- E représente un élément pouvant donner un oxyde de structure rutile ou un élément qui, associé à V, Sb, Sn, Ti, Fe et/ou à un autre élément E, peut donner une phase de structure rutile ou trirutile ou une solution solide de structure rutile
- a représente un nombre entier ou fractionnaire égal ou supérieur à 0,5
- b, c, d et e représentent indépendamment les uns des autres un nombre entier ou fractionnaire de 0 à 100
- x représente un nombre entier ou fractionnaire déterminé par le degré d'oxydation des autres éléments,
et un support solide de type oxyde,
ledit procédé étant caractérisé en ce qu'il comprend :
- l'imprégnation du support solide par une solution dans au moins un alcool saturé de composés respectifs de vanadium, d'antimoine et éventuellement d'étain et/ou de titane et/ou de fer et/ou d'élément E,
- la mise en contact du support solide imprégné obtenu, avec une solution aqueuse tampon à pH compris entre 6 et 8,
- la séparation du solide et son séchage,
- la calcination du solide en deux étapes, tout d'abord à une température de 300°C à 350°C, puis à une température de 400°C à 800°C.

2. Procédé selon la revendication 1, caractérisé en ce que le support solide est un oxyde tel qu'une alumine, une silice, une silice-alumine, une zircone, une cérine, un oxyde mixte de cérium et de zirconium, une magnésie, un oxyde de titane, un oxyde de niobium, un oxyde de lanthane.

3. Procédé selon l'une des revendications 1 ou 2, caractérisé en ce que le support solide présente une taille de particules de 10 µm à 1000 µm et de préférence de 20 µm à 300 µm.

4. Procédé selon l'une des revendications 1 à 3, caractérisé en ce que le support solide présente un volume poreux d'au moins 0,1 cm³/g et de préférence d'au moins 0,15 cm³/g.

5. Procédé selon l'une des revendications 1 à 4, caractérisé en ce que dans le catalyseur final, le rapport pondéral entre la phase active de formule (I) et la totalité dudit catalyseur est de 5 à 50 %.

6. Procédé selon l'une des revendications 1 à 5, caractérisé en ce que le composé soluble du vanadium est choisi parmi l'acétylacétonate de vanadyle, le trichlorure de vanadyle, le trifluorure de vanadium, le tétrafluorure de vanadium, le pentafluorure de vanadium, le tribromure de vanadium, le dichlorure de vanadium, le trichlorure de vanadium, le tétrachlorure de vanadium, le triiodure de vanadium.

7. Procédé selon l'une des revendications 1 à 6, caractérisé en ce que le composé soluble de l'antimoine est choisi parmi le pentachlorure d'antimoine, le trichlorure d'antimoine, le tribromure d'antimoine, le trifluorure d'antimoine, le triiodure d'antimoine, le trioxyde d'antimoine, l'hydrure d'antimoine.

8. Procédé selon l'une des revendications 1 à 7, caractérisé en ce que le composé soluble de l'étain est choisi parmi le chlorure stannique, le chlorure stanneux, le bromure stanneux.

9. Procédé selon l'une des revendications 1 à 8, caractérisé en ce que le composé soluble du titane est choisi parmi le dichlorure de titane, le tétrachlorure de titane, le trichlorure de titane, le tribromure de titane, le tétrabromure de titane, le tétrafluorure de titane, le diiodure de titane.

10. Procédé selon l'une des revendications 1 à 9, caractérisé en ce que le composé soluble du fer est choisi parmi le dichlorure de fer, le trichlorure de fer, le dibromure de fer, le tribromure de fer, le diiodure de fer, le nitrate ferreux, le sulfate ferreux, le sulfate ferrique, le thiosulfate ferreux, le formiate ferrique, l'acétate ferrique, l'acétylacétonate ferrique, le benzoate ferrique, l'oléate ferrique, le lactate ferreux, le lactate ferrique.

11. Procédé selon l'une des revendications 1 à 10, caractérisé en ce que l'alcool saturé mis en oeuvre est choisi parmi les alcanols et les cycloalcanols et de préférence, les alcanols ayant de 1 à 6 atomes de carbone, tels que le méthanol, l'éthanol, le n.propanol, le propanol-2, le n.butanol, le butanol-2, le tertiobutanol, les pentanols et les hexanols, et le cyclohexanol.

12. Procédé selon l'une des revendications 1 à 11, caractérisé en ce que l'imprégnation du support solide par la solution alcoolique des composés des métaux entrant dans la composition de la phase active de formule (I) est effectuée en mettant en oeuvre un volume de solution alcoolique inférieur ou égal au volume poreux total du support, ledit volume de la solution alcoolique représentant de préférence au moins 50 % dudit volume poreux total.

13. Procédé selon l'une des revendications 1 à 12, caractérisé en ce que le support solide imprégné est mis en contact avec une solution aqueuse tampon d'un sel d'ammonium, comportant éventuellement de l'ammoniaque, le sel d'ammonium utilisé étant préférentiellement un carboxylate d'ammonium, le carbonate d'ammonium, l'hydrogénophosphate d'ammonium.

14. Procédé selon l'une des revendications 1 à 13, caractérisé en ce que le volume de la solution tampon mise en contact avec le support imprégné est égal ou supérieur au volume poreux total du support et est de préférence supérieur au volume poreux total du support.

15. Procédé selon l'une des revendications 1 à 14, caractérisé en ce que la phase active répond à la formule (I) dans laquelle :
- E représente un ou plusieurs éléments choisis parmi le nickel, le gallium, l'aluminium, le niobium
- a représente un nombre entier ou fractionnaire égal ou inférieur à 100 et compris de préférence entre 0,5 et 50
- b, c, d et e représentent indépendamment les uns des autres un nombre entier ou fractionnaire de 0 à 50, l'un au moins de ces symboles étant supérieur à 0 et de préférence égal ou supérieur à 0,5
- x représente un nombre entier ou fractionnaire déterminé par le degré d'oxydation des autres éléments.

16. Procédé d'ammoxydation d'alcanes en phase vapeur dans un réacteur à lit fluidisé ou à lit transporté en présence d'un catalyseur solide, caractérisé en ce que ledit catalyseur est obtenu par le procédé selon l'une des revendications 1 à 15.

17. Procédé selon la revendication 16, caractérisé en ce que des alcanes ayant de 3 à 12 atomes de carbone par molécule, choisis de préférence parmi le propane et l'isobutane, sont mis à réagir en phase vapeur avec de l'ammoniac et de l'oxygène.

18. Procédé selon l'une des revendications 16 ou 17, caractérisé en ce que au sein du gaz réactif, constitué par l'alcane, l'ammoniac, l'oxygène, la teneur volumique en alcane est de préférence comprise entre 5 et 70 %, la teneur volumique en ammoniac est de préférence comprise entre 3 et 50 % et la teneur volumique en oxygène est de préférence comprise entre 3 et 45 %.

19. Procédé selon l'une des revendications 16 à 18, caractérisé en ce que la température de réaction est comprise entre 300°C et 550°C et, de préférence, entre 400°C et 500°C.

## Claims

1. Process for the preparation of a catalyst for a fluidized bed or moving bed reactor comprising an active phase of general empirical formula (I) :
VSbₐSn_{b}Ti_{c}Fe_{d}EₑOₓ (I)
in which:
- E represents an element which can give an oxide of rutile structure or an element which, in combination with V, Sb, Sn, Ti, Fe and/or with another element E, can give a phase of rutile or trirutile structure or a solid solution of rutile structure
- a represents a whole or fractional number equal to or greater than 0.5
- b, c, d and e represent, independently of one another, a whole or fractional number from 0 to 100
- x represents a whole or fractional number determined by the oxidation number of the other elements,
and a solid support of oxide type,
the said process being characterized in that it comprises:
- the impregnation of the solid support by a solution in at least one saturated alcohol of respective compounds of vanadium, of antimony and optionally of tin and/or of titanium and/or of iron and/or of element E,
- bringing the impregnated solid support obtained into contact with an aqueous buffer solution at a pH of between 6 and 8,
- the separation of the solid and the drying thereof,
- the calcination of the solid in two stages, first of all at a temperature of 300°C to 350°C and then at a temperature of 400°C to 800°C.

2. Process according to Claim 1, characterized in that the solid support is an oxide, such as an alumina, a silica, a silica/alumina, a zirconia, a cerite, a mixed oxide of cerium and of zirconium, a magnesia, a titanium oxide, a niobium oxide or a lanthanum oxide.

3. Process according to either of Claims 1 and 2, characterized in that the solid support has a particle size from 10 µm to 1,000 µm and preferably from 20 µm to 300 µm.

4. Process according to one of Claims 1 to 3, characterized in that the solid support exhibits a pore volume of at least 0.1 cm³/g and preferably of at least 0.15 cm³/g.

5. Process according to one of Claims 1 to 4, characterized in that the ratio by weight in the final catalyst of the active phase of formula (I) to the whole of the said catalyst is from 5 to 50 %.

6. Process according to one of Claims 1 to 5, characterized in that the soluble vanadium compound is chosen from vanadyl acetylacetonate, vanadyl trichloride, vanadium trifluoride, vanadium tetrafluoride, vanadium pentafluoride, vanadium tribromide, vanadium dichloride, vanadium trichloride, vanadium tetrachloride or vanadium triiodide.

7. Process according to one of Claims 1 to 6, characterized in that the soluble antimony compound is chosen from antimony pentachloride, antimony trichloride, antimony tribromide, antimony trifluoride, antimony triiodide, antimony trioxide or stibine.

8. Process according to one of Claims 1 to 7, characterized in that the soluble tin compound is chosen from stannic chloride, stannous chloride or stannous bromide.

9. Process according to one of Claims 1 to 8, characterized in that the soluble titanium compound is chosen from titanium dichloride, titanium tetrachloride, titanium trichloride, titanium tribromide, titanium tetrabromide, titanium tetrafluoride or titanium diiodide.

10. Process according to one of Claims 1 to 9, characterized in that the soluble iron compound is chosen from iron dichloride, iron trichloride, iron dibromide, iron tribromide, iron diiodide, ferrous nitrate, ferrous sulphate, ferric sulphate, ferrous thiosulphate, ferric formate, ferric acetate, ferric acetylacetonate, ferric benzoate, ferric oleate, ferrous lactate or ferric lactate.

11. Process according to one of Claims 1 to 10, characterized in that the saturated alcohol used is chosen from alkanols and cycloalkanols and preferably alkanols having from 1 to 6 carbon atoms, such as methanol, ethanol, n-propanol, 2-propanol, n-butanol, 2-butanol, tert-butanol, pentanols and hexanols, and cyclohexanol.

12. Process according to one of Claims 1 to 11, characterized in that the impregnation of the solid support by the alcoholic solution of the compounds of the metals which form part of the composition of the active phase of formula (I) is carried out by using a volume of alcoholic solution which is less than or equal to the total pore volume of the support, the said volume of the alcoholic solution preferably representing at least 50 % of the said total pore volume.

13. Process according to one of Claims 1 to 12, characterized in that the impregnated solid support is brought into contact with an aqueous buffer solution of an ammonium salt, optionally containing aqueous ammonia, the ammonium salt used preferentially being an ammonium carboxylate, ammonium carbonate or ammonium hydrogenphosphate.

14. Process according to one of Claims 1 to 13, characterized in that the volume of the buffer solution brought into contact with the impregnated support is equal to or greater than the total pore volume of the support and is preferably greater than the total pore volume of the support.

15. Process according to one of Claims 1 to 14, characterized in that the active phase corresponds to the formula (I) in which:
- E represents one or a number of elements chosen from nickel, gallium, aluminium or niobium
- a represents a whole or fractional number equal to or less than 100 and preferably of between 0.5 and 50
- b, c, d and e represent, independently of one another, a whole or fractional number from 0 to 50, at least one of these symbols being greater than 0 and preferably equal to or greater than 0.5
- x represents a whole or fractional number determined by the oxidation number of the other elements.

16. Process for the vapour-phase ammoxidation of alkanes in a fluidized bed or moving bed reactor in the presence of a solid catalyst, characterized in that the said catalyst is obtained by the process according to one of Claims 1 to 15.

17. Process according to Claim 16, characterized in that alkanes having from 3 to 12 carbon atoms per molecule, preferably chosen from propane and isobutane, are reacted in the vapour phase with ammonia and oxygen.

18. Process according to either of Claims 16 and 17, characterized in that, within the reactive gas consisting of the alkane, ammonia and oxygen, the alkane content by volume is preferably between 5 and 70 %, the ammonia content by volume is preferably between 3 and 50 % and the oxygen content by volume is preferably between 3 and 45 %.

19. Process according to one of Claims 16 to 18, characterized in that the reaction temperature is between 300°C and 550°C and preferably between 400°C and 500°C.

## Patentansprüche

1. Verfahren zur Herstellung eines Katalysators für einen Reaktor mit Wirbelbett oder Förderbett, umfassend eine aktive Phase der allgemeinen empirischen Formel (I):
V Sbₐ Sn_{b} Ti_{c} Fe_{d} Eₑ Oₓ (I)
worin:
- E ein Element, das ein Oxid mit Rutilstruktur ergeben kann, oder ein Element, das in Assoziation mit V, Sb, Sn, Ti, Fe und/oder einem weiteren Element E eine Phase mit Rutil- oder Trirutilstruktur oder eine feste Lösung mit Rutilstruktur ergeben kann, wiedergibt;
- a eine ganze Zahl oder eine Bruchzahl entsprechend oder größer als 0,5 bedeutet;
- b, c, d und e unabhängig voneinander eine ganze Zahl oder eine Bruchzahl von 0 bis 100 bedeuten;
- x eine ganze Zahl oder eine Bruchzahl, bestimmt durch den Oxidationsgrad der anderen Elemente, bedeutet
und einen festen Träger vom Oxidtyp,
wobei dieses Verfahren dadurch gekennzeichnet ist, daß es umfaßt:
- die Imprägnierung des festen Trägers mit einer Lösung in zumindest einem gesättigten Alkohol der jeweiligen Verbindungen von Vanadin, Antimon und gegebenenfalls Zinn und/oder Titan und/oder Eisen und/oder einem Element E;
- das Inkontaktbringen des erhaltenen imprägnierten festen Trägers mit einer wässrigen Pufferlösung mit einem pH zwischen 6 und 8;
- die Abtrennung des Feststoffs und seine Trocknung;
- die Kalzinierung des Feststoffs in zwei Stufen, zunächst bei einer Temperatur von 300 bis 350°C, dann bei einer Temperatur von 400 bis 800°C.

2. Verfahren nach Anspruch 1, dadurch gekennzeichnet, daß der feste Träger ein Oxid, wie ein Aluminiumoxid, ein Siliciumdioxid, ein Siliciumdioxid-Aluminiumoxid, ein Zirkonoxid, ein Ceroxid, ein gemischtes Oxid von Cer und Zirkonium, ein Magnesiumoxid, ein Titanoxid, ein Nioboxid, ein Lanthanoxid, ist.

3. Verfahren gemäß einem der Ansprüche 1 oder 2, dadurch gekennzeichnet, daß der feste Träger eine Teilchengröße von 10 bis 1000 µm, und vorzugsweise von 20 bis 300 µm, besitzt.

4. Verfahren gemäß einem der Ansprüche 1 bis 3, dadurch gekennzeichnet, daß der feste Träger ein Porenvolumen von zumindest 0,1 cm³/g, und vorzugsweise von zumindest 0,15 cm³/g, besitzt.

5. Verfahren gemäß einem der Ansprüche 1 bis 4, dadurch gekennzeichnet, daß in dem fertiggestellten Katalysator das Gewichtsverhältnis zwischen der aktiven Phase der Formel 1 und der Gesamtheit des Katalysators 5 bis 50% beträgt.

6. Verfahren gemäß einem der Ansprüche 1 bis 5, dadurch gekennzeichnet, daß die lösliche Verbindung des Vanadins ausgewählt wird unter Vanadylacetylacetonat, Vanadyltrichlorid, Vanadintrifluorid, Vanadintetrafluorid, Vanadinpentafluorid, Vanadintribromid, Vanadindichlorid, Vanadintrichlorid, Vanadintetrachlorid, Vanadintrijodid.

7. Verfahren gemäß einem der Ansprüche 1 bis 6, dadurch gekennzeichnet, daß die lösliche Verbindung des Antimons unter Antimonpentachlorid, Antimontrichlorid, Antimontribromid, Antimontrifluorid, Antimontrijodid, Antimontrioxid, Antimonhydrid, ausgewählt wird.

8. Verfahren gemäß einem der Ansprüche 1 bis 7, dadurch gekennzeichnet, daß die lösliche Verbindung des Zinn unter Stannichlorid, Stannochlorid, Stannobromid, ausgewählt wird.

9. Verfahren gemäß einem der Ansprüche 1 bis 8, dadurch gekennzeichnet, daß die lösliche Verbindung des Titans unter Titandichlorid, Titantetrachlorid, Titantrichlorid, Titantribromid, Titantetrabromid, Titantetrafluorid, Titandijodid, ausgewählt wird.

10. Verfahren gemäß einem der Ansprüche 1 bis 9, dadurch gekennzeichnet, daß die lösliche Verbindung des Eisens unter Eisendichlorid, Eisentrichlorid, Eisendibromid, Eisentribromid, Eisendijodid, Ferronitrat, Ferrosulfat, Ferrisulfat, Ferrothiosulfat, Ferriformiat, Ferriacetat, Ferriacetylacetonat, Ferribenzoat, Ferrioleat, Ferrolactat, Ferrilactat, ausgewählt wird.

11. Verfahren gemäß einem der Ansprüche 1 bis 10, dadurch gekennzeichnet, daß der eingesetzte gesättigte Alkohol unter den Alkanolen und den Cycloalkanolen, und vorzugsweise den Alkanolen mit 1 bis 6 Kohlenstoffatomen, wie Methanol, Ethanol, n-Propanol, 2-Propanol, n-Butanol, 2-Butanol, tert.-Butanol, den Pentanolen und den Hexanolen und Cyclohexanol ausgewählt wird.

12. Verfahren gemäß einem der Ansprüche 1 bis 11, dadurch gekennzeichnet, daß die Imprägnierung des festen Trägers durch die alkoholische Lösung der Metallverbindungen, die in die Zusammensetzung der aktiven Phase der Formel (I) eintreten, bewirkt wird, indem man ein Volumen an alkoholischer Lösung, das niedriger ist als oder gleich ist dem gesamten Porenvolumen des Trägers, einsetzt, wobei dieses Volumen der alkoholischen Lösung vorzugsweise zumindest 50% des gesamten Porenvolumens beträgt.

13. Verfahren gemäß einem der Ansprüche 1 bis 12, dadurch gekennzeichnet, daß der imprägnierte feste Träger mit einer wässrigen Pufferlösung eines Ammoniumsalzes, die gegebenenfalls Ammoniak enthält, in Kontakt gebracht wird, wobei das verwendete Ammoniumsalz vorzugsweise ein Amimoniumcarboxylat, Ammoniumcarbonat, Ammoniumhydrogenphosphat, ist.

14. Verfahren gemäß einem der Ansprüche 1 bis 13, dadurch gekennzeichnet, daß das Volumen der mit dem imprägnierten Träger in Kontakt gebrachten Pufferlösung gleich ist dem oder größer ist als das gesamte Porenvolumen des Trägers, und vorzugsweise größer ist als das gesamte Porenvolumen des Trägers.

15. Verfahren gemäß einem der Anspruche 1 bis 14, dadurch gekennzeichnet, daß die aktive Phase der Formel (I) entspricht, worin:
- E eines oder mehrere Elemente, ausgewählt unter Nickel, Gallium, Aluminium, Niob, bedeutet;
- a eine ganze Zahl oder eine Bruchzahl gleich oder geringer als 100 bedeutet und vorzugsweise zwischen 0,5 und 50 liegt;
- b, c, d und e unabhängig voneinander eine ganze Zahl oder eine Bruchzahl von 0 bis 50 bedeuten, wobei zumindest eines dieser Symbole größer als 0 und vorzugsweise gleich oder größer als 0,5 ist;
- x eine ganze Zahl oder Bruchzahl wiedergibt, die von dem Oxidationsgrad der anderen Elemente bestimmt ist.

16. Verfahren zur Ammoxidation von Alkanen in der Gasphase in einem Reaktor mit Wirbelbett oder Förderbett in Gegenwart eines festen Katalysators, dadurch gekennzeichnet, daß der Katalysator nach dem Verfahren gemäß einem der Ansprüche 1 bis 15 erhalten wird.

17. Verfahren gemäß Anspruch 16, dadurch gekennzeichnet, daß Alkane mit 3 bis 12 Kohlenstoffatomen je Molekül, vorzugsweise ausgewählt unter Propan und Isobutan, in der Gasphase mit Ammoniak und Sauerstoff zur Reaktion gebracht werden.

18. Verfahren gemäß einem der Ansprüche 16 oder 17, dadurch gekennzeichnet, daß innerhalb des Reaktionsgases, bestehend aus Alkan, Ammoniak, Sauerstoff, der Volumengehalt an Alkan vorzugsweise zwischen 5 und 70%, der Volumengehalt an Ammoniak vorzugsweise zwischen 3 und 50%, und der Volumengehalt an Sauerstoff vorzugsweise zwischen 3 und 45% liegt.

19. Verfahren gemäß einem der Ansprüche 16 bis 18, dadurch gekennzeichnet, daß die Reaktionstemperatur zwischen 300 und 550°C, und vorzugsweise zwischen 400 und 500°C liegt.
